# FASCICULE DE BREVET EUROPEEN

(11) **EP 1 906 848 B1**
(45) Date de publication et mention de la délivrance du brevet: **30.12.2015**
(21) Numéro de dépôt: 06794407.4
(22) Date de dépôt: 09.05.2006
(51) Int. Cl.: A61B 17/34, A61B 17/88

(54) **SERINGUE POUR BIOMATÉRIAU**
SPRITZE FÜR BIOMATERIAL
SYRINGE FOR BIOMATERIAL

(30) Priorité: 10.05.2005 FR 0504659
(43) Date de publication de la demande: 09.04.2008
(73) Titulaire: BIO HOLDINGS INTERNATIONAL LIMITED, Tortola (VG)
(72) Inventeur: NASSIRI, Nasser, F-92400 Courbevoie (FR); CIROTTEAU, Yves, F-75007 Paris (FR); JUSSMANN, Alberto, F-92160 Antony (FR)
(74) Mandataire: Intès, Didier Gérard André
(86) Numéro de dépôt international: PCT/FR2006/050422
(87) Numéro de publication internationale: WO 2007/003799

(56) Documents cités:
- WO-A-01/56515
- WO-A-03/037165
- FR-A- 2 820 631
- US-A- 5 752 940
- US-A1- 2001 021 852
- US-B1- 6 436 143

## Description

La présente invention a pour objet un dispositif d'injection percutané de type seringue destiné à l'injection percutané d'un biomatériau sous forme de particules (de tailles différentes) ou de pâte.

De façon plus précise, l'invention concerne un dispositif qui permet d'injecter, notamment à l'intérieur d'un os un biomatériau tel que par exemple du corail naturel ou d'autres matériaux analogues - tel par exemple tous les sels de calcium utilisés seuls ou mélangés à d'autres substances : BMP, extraits de moelle osseuse, cellules ostéogènes autologues ou de culture, ciments etc... se présentant sous forme de particules de petite dimension ou de pâte.

Le produit peut être injecté sous forme de poudre de particules ou de pâte.

Dans certaines pathologies de l'os, congénitales ou acquises, la partie externe de l'os conserve son intégrité et ses propriétés mécaniques. Dans d'autres et en particulier des maladies liées à la déminéralisation ou à des défauts de minéralisation de l'os, elle subit ainsi que la partie interne, des phénomènes de dégénérescence qui peuvent entraîner la formation à l'intérieur de l'os de cavités de dimensions de taille différente plus ou moins importantes. Même si cette situation n'entraîne pas immédiatement un effet incapacitant pour le sujet, la présence de cavités intra osseuses diminue la résistance mécanique de cet os - par exemple l'extrémité supérieure du fémur - de façon d'autant plus importante que l'étendue des dites cavités est plus grande. Cette situation peut entraîner des risques de fractures d'autant plus graves que la perte osseuse est grande.

On a déjà proposé dans le brevet européen EP 0 649 309, en ce qui concerne le traitement local des maladies de l'os liées à la déminéralisation ou à des défauts de minéralisation de l'os et en particulier la fracture du col du fémur et sa prévention qui sont liées directement à ces maladies, à titre préventif, d'intervenir pour combler à l'aide de particules de biomatériau ces cavités formées à l'intérieur de l'os afin de régénérer cette partie interne de l'os dans le but de relancer le processus de reminéralisation de celui-ci et de redonner ainsi à l'os sa résistance mécanique initiale ou une résistance proche de cette résistance mécanique initiale.

FR-A-2820631 divulge un ancillaire d'injection de substitut osseux comprenant un trocart, un réservoir sensiblement cylindrique avec un piston, ledit réservoir ayant deux extrémités ouvertes et pouvant être inséré dans la partie distale du trocart.

L'objet de cette intervention préventive est de traiter localement les maladies liées à la déminéralisation ou à des défauts de minéralisation de l'os en apportant localement un médicament (en particulier les biomatériaux tel décrits dans le brevet précédemment déposé par le groupe) bien avant que la maladie n'entraîne la survenue des fractures. Ces fractures qui surviennent plus souvent chez les personnes âgées sont très compliquées et immobilisent les patients plusieurs mois avec des conséquences importantes sur la fonction de la hanche et la vie des patients.

La technique utilisée actuellement pour réaliser cette opération consiste à percer un orifice dans la partie externe de l'os au droit de la cavité ou des cavités internes puis, à l'aide d'une spatule ou d'une curette, on introduit du mieux possible les particules de biomatériau dans l'orifice afin que ces particules comblent la cavité interne de l'os induite par la déminéralisation de cet os. Cette opération est relativement longue et délicate et il n'est pas possible d'être sûr que la cavité est effectivement totalement comblée par les particules de biomatériau.

Il existe donc un réel besoin de disposer d'un dispositif permettant simultanément la réalisation de l'orifice dans la partie externe de l'os et l'injection dans la cavité interne de l'os des particules de biomatériau.

Un objet de la présente invention est de fournir un dispositif percutané d'injection de biomatériau sous forme et selon les caractéristiques d écrites plus haut dans la présente invention qui répond aux besoins rappelés ci-dessus.

Cette injection se fait soit du côté opposé au côté de la fracture et dans le même temps opératoire que celui du traitement de la fracture, soit à titre purement préventif dans le même temps opératoire des deus côtés non fracturés, après avoir mesuré par ostéodensitométrie la perte osseuse et - par exemple avoir localisé les pertes osseuses à l'aide d'un procédé radiographique en trois dimensions. Cette intervention se fait après accord du patient selon les techniques de bonne pratique

Pour atteindre ce but, selon l'invention, le dispositif d'injection de biomatériau sous forme de particules ou de pâte est défini par la revendication 1 et comprend:
- un trocart de forme générale cylindrique présentant une première extrémité ouverte présentant un bord coupant, la paroi de ladite première extrémité présentant au moins un orifice pour le passage du biomatériau, et une deuxième extrémité également ouverte,
- un réservoir pour recevoir ledit biomatériau, comprenant une pièce de forme sensiblement cylindrique et ayant deux extrémités ouvertes, ladite pièce cylindrique pouvant être insérée dans au moins une partie du trocart par la deuxième extrémité de celui-ci et présentant un diamètre interne sensiblement constant sur toute sa longueur, et des moyens amovibles d'obturation des deux extrémités du réservoir ; et
- un piston insérable dans ladite pièce cylindrique lorsque lesdits moyens d'obturation sont enlevés, ledit piston pouvant coulisser dans ladite pièce cylindrique sur toute la longueur de celle-ci et dans la première extrémité du trocart.

On comprend que, grâce à la présence à l'extrémité du trocart du bord coupant, le dispositif d'injection permet de réaliser lui-même l'orifice dans la partie externe de l'os. De plus, le réservoir qui peut être engagé dans le trocart permet de préparer la composition à base de biomatériau qui devra être injectée dans l'os. En effet, comme on l'expliquera ultérieurement, le plus souvent, les particules de biomatériau doivent être mélangées à un autre composant. Enfin, le piston permet d'appliquer une pression suffisante au biomatériau contenu dans le réservoir pour que les particules sortant du trocart par son extrémité ouverte et par les orifices qu'il comporte assurent, grâce à cette pression, un remplissage convenable de la cavité interne induite dans l'os par la déminéralisation de l'os.

De préférence, la deuxième extrémité du trocart comporte des moyens externes tels que des encoches ou des trous pour permettre la mise en rotation du trocart autour de son axe longitudinal afin de favoriser la réalisation de l'orifice dans la partie externe de l'os.

De préférence également, le dispositif comprend en outre un embout de percussion qui peut être fixé de façon temporaire sur la deuxième extrémité du trocart avant la mise en place du réservoir pour faciliter l'effet de découpe de la partie externe de l'os à l'aide de l'extrémité coupante du trocart.

De préférence également, la paroi interne du trocart comporte à proximité de sa première extrémité un épaulement pour limiter l'enfoncement du réservoir dans le trocart. De même, la paroi externe du trocart comporte à proximité de sa première extrémité un épaulement pour limiter l'enfoncement de l'extrémité du trocart dans l'os.

De préférence encore, le diamètre interne du réservoir et le diamètre interne de la première extrémité du trocart s'étendant entre l'épaulement interne et l'extrémité et le bord coupant sont sensiblement égaux afin d'éviter la présence de singularités dans l'écoulement des particules de biomatériau lors de l'action du piston.

D'autres caractéristiques et avantages de l'invention apparaîtront mieux à la lecture de la description qui suit d'un mode de réalisation de l'invention donné à titre d'exemples non limitatifs. La description se réfère aux figures annexées, sur lesquelles :
- la figure 1A est une vue en coupe longitudinale du trocart ;
- la figure 1B est une vue de la deuxième extrémité du trocart selon un mode préféré de réalisation ;
- la figure 1C montre en vue de côté un exemple préféré de réalisation de la première extrémité du trocart ;
- la figure 2 est une vue de côté de l'embout de percussion ;
- la figure 3A est une vue en coupe longitudinale de la pièce cylindrique du réservoir ;
- les figures 3B et 3C montrent en vue de côté les moyens d'obturation qui peuvent être placés aux extrémités de la pièce cylindrique du réservoir ;
- la figure 4 est une vue de côté du piston ;
- la figure 5 montre la première phase d'utilisation du dispositif d'injection illustrant plus particulièrement la perforation dans la paroi externe de l'os et l'utilisation de l'embout de percussion ;
- la figure 6 montre le réservoir obturé à ses extrémités pour réaliser la préparation à base de biomatériau ; et
- la figure 7 montre l'ensemble du dispositif d'injection prêt pour procéder à l'introduction du biomatériau dans la cavité interne de l'os induite par la déminéralisation de l'os.

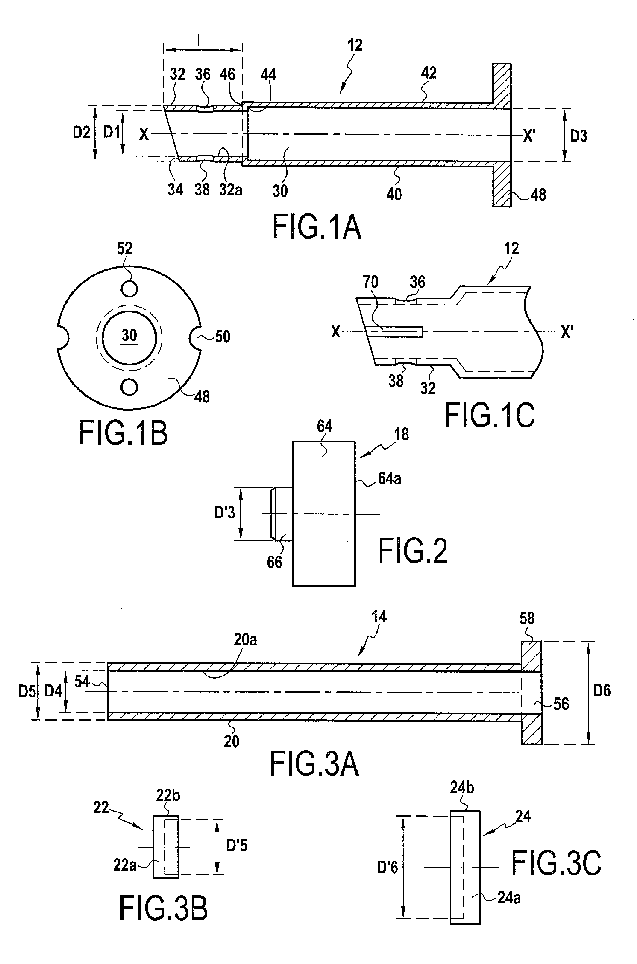

En se référant tout d'abord aux figures 1 à 4, on va décrire les différents éléments constitutifs du dispositif d'injection de biomatériau.

Ainsi qu'on l'a déjà indiqué, le dispositif d'injection est constitué essentiellement par un trocart 12, un réservoir de biomatériau 14 et un piston 16. Le dispositif d'injection comporte également de préférence un embout amovible de percussion 18 et des bouchons d'obturation de la pièce cylindrique 20 du réservoir, ces bouchons étant référencés 22 et 24.

Le trocart 12 du dispositif d'injection 10 représenté sur la figure 1A a une forme générale cylindrique avec un évidement interne 30. Le trocart 12 comporte une première extrémité 32 de diamètre externe D2 et de diamètre interne D1. Le bord 34 de la première extrémité 32 est coupant et de préférence ce bord est disposé dans un plan qui n'est pas orthogonal à l'axe X,X' du trocart. Cette première extrémité 32 comporte également dans sa paroi des orifices 36 et 38 de dimension suffisante pour permettre l'écoulement des particules de biomatériau. Le trocart 12 comporte une partie médiane 40 et une deuxième extrémité 42. Le diamètre interne de la partie médiane 40 et de la deuxième extrémité 42 référencé D3 est supérieur au diamètre interne D1 de la première extrémité 32. La paroi interne du trocart définit ainsi un épaulement interne 44. Cette différence de diamètre interne définit également un épaulement externe 46. Dans l'exemple décrit, le diamètre interne D1 de la première extrémité du trocart est égal à 12 mm. Plus généralement, ce diamètre est compris entre 9 et 15 mm. La longueur I de l'extrémité du trocart est de 20 mm dans l'exemple décrit.

La deuxième extrémité 42 du trocart comporte une collerette externe 48 dont la fonction est de permettre la manipulation du trocart éventuellement à l'aide d'un instrument ou d'un outil. Sur la figure 1B, on a représenté la collerette 48 qui peut comporter avantageusement des crans externes tels que 50 ou des orifices 52 servant à la prise d'outils de mise en rotation du trocart pour améliorer l'effet de son bord coupant 34.

Le réservoir 14 est constitué par la pièce 20 qui a une forme cylindrique avec un diamètre interne D4 et un diamètre externe D5. La première extrémité 54 de la pièce cylindrique 20 est ouverte ainsi d'ailleurs que sa deuxième extrémité 56. La deuxième extrémité 56 est munie d'une collerette externe 58. Pour permettre l'obturation du réservoir, le dispositif comporte également des bouchons d'extrémité 22 et 24. Le bouchon d'extrémité 22 destiné à coopérer avec la première extrémité 54 de la pièce cylindrique 20 présente un fond 22a et une jupe périphérique 22b définissant une cavité interne de diamètre D'5 permettant la fixation du bouchon 22 sur l'extrémité 54 par des formations élastiques. De la même manière, le deuxième bouchon d'extrémité 24 comporte un fond 24a et une jupe périphérique 24b définissant une cavité interne de diamètre D'6 de telle manière que le bouchon 24 puisse être fixé sur la collerette 58 par déformation élastique.

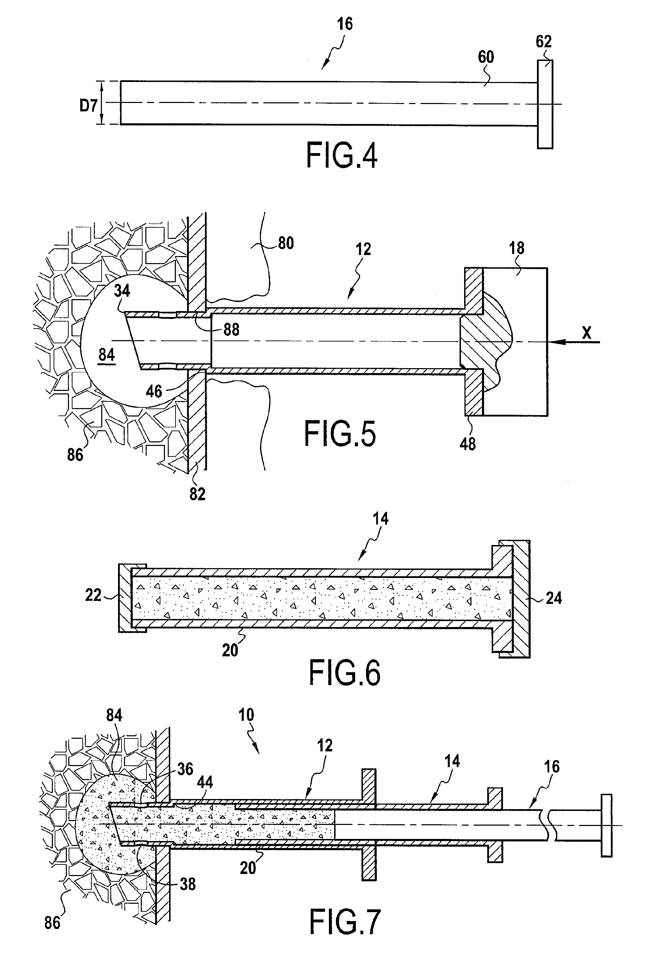

La figure 4 montre le piston ou poussoir 16 qui est plein. Il présente un diamètre externe constant D7 et, à son extrémité 60, il est équipé d'une collerette 62 pour faciliter l'action de l'opérateur sur le poussoir 16.

De préférence, le dispositif d'injection 10 comporte également un embout de percussion 18. Cet embout représenté sur la figure 2 comporte un corps principal cylindrique 64 définissant une face de percussion 64a et un prolongement de fixation 66 qui fait saillie hors du corps 64 et présente un diamètre externe D'3 légèrement supérieur au diamètre interne D3 de la deuxième extrémité du trocart de façon à permettre la fixation de l'embout de percussion 18 de façon amovible sur la deuxième extrémité du trocart.

Il faut ajouter que le diamètre D1 de la paroi interne 32a de l'extrémité 32 du trocart et le diamètre D4 de la paroi interne 20a de la pièce cylindrique 20 du réservoir sont sensiblement égaux. Ainsi, lorsque le réservoir 14 est introduit dans le trocart 12, le passage interne qui en résulte présente une paroi sensiblement constante, c'est-à-dire dépourvue d'obstacles. Il faut ajouter que, bien entendu, le diamètre externe D7 du piston 16 est légèrement inférieur au diamètre interne D4 de la pièce cylindrique 20 du réservoir de telle manière que le piston 16 puisse coulisser librement à l'intérieur de la pièce cylindrique 20 et à l'intérieur de l'extrémité 32 du trocart. Cependant, la différence entre les deux diamètres est réduite pour éviter que des particules de biomatériau ne puissent venir se coincer entre la face externe du piston 16 et la paroi interne du réservoir 14.

Sur la figure 1C, on a représenté un mode préféré de réalisation de l'extrémité 32 du trocart 12. Selon ce mode de réalisation, la face externe de l'extrémité 32 peut être équipée de lames telles que 70 qui s'étendent parallèlement à l'axe X,X' du trocart. Cette lame 70 ou ces lames 70 sont bien sûr disposées entre les orifices 36 et 38. Elle permet de "gratter" la paroi de l'orifice réalisé à l'aide du trocart en faisant tourner celle-ci.

En se référant maintenant aux figures 5 à 7, on va décrire l'utilisation du dispositif d'injection percutané d'injection de biomatériau.

Dans un premier temps, le chirurgien repère; à l'aide d'un amplificateur de brillance, le point d'entrée du trocart 12. Il fait au bistouri une petite incision cutanée pour permettre l'introduction de l'instrument. Sous amplificateur de brillance, il traverse la partie externe de l'os dans laquelle il souhaite injecter le biomatériau. C'est-à-dire derrière laquelle une cavité 84 s'est formée dans la partie interne 86 de l'os. Pour réaliser un orifice 88 dans la paroi externe de l'os 82, le chirurgien utilise bien sûr l'extrémité coupante 34 du trocart 12. Pour cela, il peut s'aider d'un outil qui coopère avec les crans 50 ou les orifices 52 prévus dans la collerette 48 du trocart de manière à imprimer au trocart un mouvement de rotation autour de son axe longitudinal X,X'. Le chirurgien peut également mettre en place l'embout de percussion 18 pour permettre à l'aide d'un instrument contondant d'exercer une pression plus importante sur l'os à l'aide du bord coupant 34 du trocart.

Lorsque l'orifice 88 a été totalement réalisé, le trocart 12 se retrouve en appui sur l'os externe par l'épaulement externe 46 et l'extrémité 32 du trocart pénètre à l'intérieur de la cavité 84 qu'il faut traiter. Par ailleurs, le chirurgien prépare dans le réservoir 14 la composition à base de biomatériau qu'il doit utiliser. Cette composition consiste le plus souvent dans une certaine quantité de particules de biomatériau, telle que du corail naturel auquel il ajoute, par exemple, une certaine quantité de moelle osseuse pour favoriser la régénérescence de la partie interne de l'os à l'aide du biomatériau. Pour cela, il met en place successivement aux extrémités de la pièce cylindrique 20 les bouchons 22 et 24, ce qui lui permet d'agiter suffisamment l'ensemble pour obtenir un mélange parfaitement homogène.

Lorsque cette opération a été réalisée, le chirurgien enlève bien sûr les bouchons 22 et 24 du réservoir 14 et met en place la pièce cylindrique 20 à l'intérieur du trocart 12 jusqu'à ce que l'extrémité 54 de la pièce cylindrique 20 vienne en butée sur l'épaulement interne 44 du trocart. Le réservoir 14 contenant la composition à base de biomatériau est ainsi fermement maintenu dans le trocart 12. Il suffit alors d'insérer à l'extrémité de la pièce cylindrique 20 le piston 16 pour provoquer la sortie des particules de biomatériau par l'extrémité ouverte du trocart et par ses orifices latéraux 36 et 38. La pression exercée sur la composition à base de biomatériau à l'aide du piston 16 permet d'assurer que le biomatériau va convenablement remplir la cavité 84 dans la partie interne de l'os. En outre, le fait que la première extrémité du trocart soit ouverte et que cette première extrémité comporte en outre des orifices latéraux 36 et 38 favorise la sortie du biomatériau hors du trocart à l'intérieur de la cavité 84.

Dans la description précédente, on a plus particulièrement considéré le cas où le biomatériau se présente sous forme de particules. Le dispositif peut également être utilisé pour un biomatériau sous forme de pâte dès lors que sa viscosité est convenable.

## Revendications

1. Dispositif d'injection percutané de biomatériau sous forme de particules de tailles différentes ou de pâte, comprenant :
- un trocart (12) de forme générale cylindrique présentant une première extrémité ouverte (32) présentant un bord coupant (34), la paroi de ladite première extrémité présentant au moins un orifice (36, 38) pour le passage du biomatériau, et une deuxième extrémité (42) également ouverte ;
- un réservoir (14) pour recevoir ledit biomatériau, comprenant une pièce de forme sensiblement cylindrique (20) ayant deux extrémités ouvertes (54, 56), ladite pièce cylindrique pouvant être insérée dans au moins une partie du trocart par la deuxième extrémité de celui-ci et présentant un diamètre interne (D4) sensiblement constant sur toute sa longueur, le diamètre (D1) de la paroi interne (32a) de la première extrémité ouverte (32) du trocart (12) et le diamètre (D4) de la paroi interne de la pièce cylindrique (20) du réservoir (14) étant sensiblement égaux, et des moyens amovibles d'obturation des deux extrémités du réservoir (22, 24) ; et
- un piston (16) insérable dans ladite pièce cylindrique lorsque lesdits moyens d'obturation sont enlevés, ledit piston pouvant coulisser dans ladite pièce cylindrique sur toute la longueur de celle-ci et dans la première extrémité du trocart.

2. Dispositif selon la revendication 1, **caractérisé en ce que** la deuxième extrémité du trocart (12) comporte des moyens externes (48) pour permettre la mise en rotation du trocart autour de son axe longitudinal.

3. Dispositif selon la revendication 2, **caractérisé en ce que** les moyens pour faciliter la mise en rotation du trocart (12) comprennent une collerette externe (48) disposée à la deuxième extrémité (42) du trocart, ladite collerette comprenant des moyens (50, 52) pour coopérer avec un outil de mise en rotation.

4. Dispositif selon l'une quelconque des revendications 1 à 3, **caractérisé en ce qu'**il comprend en outre un embout de percussion (18) apte à être fixé de façon amovible sur la deuxième extrémité (42) du trocart (12) lorsque ledit réservoir (14) n'y est pas inséré.

5. Dispositif selon l'une quelconque des revendications 1 à 4, **caractérisé en ce que** la paroi interne du trocart (12) comprend, à proximité de sa première extrémité (32), une butée (44) pour limiter l'enfoncement de la pièce cylindrique du réservoir.

6. Dispositif selon la revendication 5, **caractérisé en ce que** ladite butée est un épaulement (44) dans la paroi interne du trocart.

7. Dispositif selon la revendication 6, **caractérisé en ce que** le diamètre interne de la pièce cylindrique (20) du réservoir (14) est sensiblement égal au diamètre interne de la partie dudit trocart (12) s'étendant entre ledit épaulement interne (44) et ledit bord coupant (34).

8. Dispositif selon la revendication 7, **caractérisé en ce que** la paroi externe du trocart (12) comporte un épaulement (46), correspondant audit épaulement interne (44), pour limiter l'enfoncement du trocart.

9. Dispositif selon l'une quelconque des revendications 1 à 8, **caractérisé en ce que** la face externe de la première extrémité (32) du trocart (12) comprend au moins une lame (70) parallèle à l'axe du trocart.

10. Dispositif selon l'une quelconque des revendications 1 à 9, **caractérisé en ce que** le bord coupant (34) de la première extrémité du trocart (12) est disposé dans un plan non orthogonal à l'axe longitudinal du trocart.

11. Dispositif selon l'une quelconque des revendications 1 à 10, **caractérisé en ce que** le diamètre interne de la première extrémité (32) du trocart (12) est compris entre 9 et 15 mm.

## Patentansprüche

1. Perkutane Injektionsvorrichtung für Biomaterial in Form von Partikeln unterschiedlicher Größen oder einer Paste, umfassend:
- einen Trokar (12) mit allgemein zylindrischer Form, der ein offenes erstes Ende (32), welches einen Schneiderand (34) aufweist, wobei die Wand des ersten Endes wenigstens eine Öffnung (36, 38) für den Durchgang des Biomaterials aufweist, sowie ein ebenfalls offenes zweites Ende (42) aufweist,
- einen Behälter (14) für die Aufnahme des Biomaterials, umfassend ein Teil mit im Wesentlichen zylindrischer Form (20), das zwei offene Enden (54, 56) aufweist, wobei das zylindrische Teil in wenigstens einen Teil des Trokars über dessen zweites Ende eingeführt werden kann und einen über seine gesamte Länge im Wesentlichen konstanten Innendurchmesser (D4) aufweist, wobei der Durchmesser (D1) der Innenwand (32a) des offenen ersten Endes (32) des Trokars (12) und der Durchmesser (D4) der Innenwand des zylindrischen Teils (20) des Behälters (14) im Wesentlichen gleich sind, sowie lösbare Mittel zum Verschließen der beiden Enden des Behälters (22, 24), und
- einen Kolben (16), der in das zylindrische Teil einsetzbar ist, wenn die Verschlussmittel entfernt sind, wobei der Kolben in dem zylindrischen Teil über dessen gesamte Länge und in dem ersten Ende des Trokars gleiten kann.

2. Vorrichtung nach Anspruch 1, **dadurch gekennzeichnet, dass** das zweite Ende des Trokars (12) äußere Mittel (48) umfasst, um das Indrehungversetzen des Trokars um seine Längsachse zu ermöglichen.

3. Vorrichtung nach Anspruch 2, **dadurch gekennzeichnet, dass** die Mittel zum Erleichtern des Indrehungversetzens des Trokars (12) einen Außenkragen (48), der an dem zweiten Ende (42) des Trokars angeordnet ist, umfassen, wobei der Kragen Mittel (50, 52) umfasst, um mit einem Werkzeug zum Indrehungversetzen zusammenzuwirken.

4. Vorrichtung nach einem der Ansprüche 1 bis 3, **dadurch gekennzeichnet, dass** sie ferner ein Schlagansatzstück (18) umfasst, das geeignet ist, an dem zweiten Ende (42) des Trokars (12) lösbar befestigt zu werden, wenn der Behälter (14) nicht darin eingesetzt ist.

5. Vorrichtung nach einem der Ansprüche 1 bis 4, **dadurch gekennzeichnet, dass** die Innenwand des Trokars (12) in der Nähe seines ersten Endes (32) einen Anschlag (44) umfasst, um das Eindringen des zylindrischen Teils des Behälters zu begrenzen.

6. Vorrichtung nach Anspruch 5, **dadurch gekennzeichnet, dass** der Anschlag eine Schulter (44) in der Innenwand des Trokars ist.

7. Vorrichtung nach Anspruch 6, **dadurch gekennzeichnet, dass** der Innendurchmesser des zylindrischen Teils (20) des Behälters (14) im Wesentlichen gleich dem Innendurchmesser des Teils des Trokars (12) ist, welcher sich zwischen der Innenschulter (44) und dem Schneiderand (34) erstreckt.

8. Vorrichtung nach Anspruch 7, **dadurch gekennzeichnet, dass** die Außenwand des Trokars (12) eine der Innenschulter (44) entsprechende Schulter (46) umfasst, um das Eindringen des Trokars zu begrenzen.

9. Vorrichtung nach einem der Ansprüche 1 bis 8, **dadurch gekennzeichnet, dass** die Außenseite des ersten Endes (32) des Trokars (12) wenigstens eine zu der Achse des Trokars parallele Klinge (70) umfasst.

10. Vorrichtung nach einem der Ansprüche 1 bis 9, **dadurch gekennzeichnet, dass** der Schneiderand (34) des ersten Endes des Trokars (12) in einer zu der Längsachse des Trokars nicht orthogonalen Ebene angeordnet ist.

11. Vorrichtung nach einem der Ansprüche 1 bis 10, **dadurch gekennzeichnet, dass** der Innendurchmesser des ersten Endes (32) des Trokars (12) zwischen 9 und 15 mm beträgt.

## Claims

1. An injector device for percutaneously injecting biomaterial in the form of particles of different sizes or of a paste, comprising:
· a trocar (12) of generally cylindrical shape presenting an open first end (32) with a sharp edge (34), the wall of said first end presenting at least one orifice (36, 38) for passing the biomaterial, and a second end (42) that is likewise open;
· a reservoir (14) for receiving said biomaterial, the reservoir comprising a part (20) of substantially cylindrical shape having two open ends (54, 56), said cylindrical part being capable of being inserted at least in a portion of the trocar via the second end thereof and presenting an inside diameter (D4) that is substantially constant over its entire length, the diameter (D1) of the inner wall (32a) of the open first end (32) of the trocar (12) and the diameter (D4) of the inner wall of the cylindrical part (20) of the reservoir (14) being substantially equal, and removable means (22, 24) for closing both ends of the reservoir; and
· a piston (16) insertable in said cylindrical part when said closure means are removed, said piston being capable of sliding in said cylindrical part over the entire length thereof and in the first end of the trocar.

2. A device according to claim 1, **characterized in that** the second end of the trocar (12) includes external means (48) for enabling the trocar to be turned about its longitudinal axis.

3. A device according to claim 2, **characterized in that** the means for facilitating turning the trocar (12) comprise an outer collar (46) disposed at the second end (42) of the trocar, said collar including means (50, 52) for co-operating with a turning tool.

4. A device according to any one of claims 1 to 3, **characterized in that** it further includes a percussion endpiece (18) suitable for being releasably fastened on the second end (42) of the trocar (12) when said reservoir (14) is not inserted therein.

5. A device according to any one of claims 1 to 4, **characterized in that** the inner wall of the trocar (12) includes, close to its first end (32), an abutment (42) for limiting the extent to which the cylindrical part of the reservoir can be engaged.

6. A device according to claim 5, **characterized in that** said abutment is a shoulder (42) in the inner wall of the trocar.

7. A device according to claim 6, **characterized in that** the inner diameter of the cylindrical part (20) of the reservoir (14) is substantially equal to the inner diameter of the portion of said trocar (12) that extends between said inner shoulder (42) and said sharp edge (34).

8. A device according to claim 7, **characterized in that** the outer wall of the trocar (12) includes a shoulder (46) corresponding to said inner shoulder (42), to limit the extent to which the trocar can be engaged.

9. A device according to any one of claims 1 to 8, **characterized in that** the outer face of the first end (32) of the trocar (12) includes at least one blade (70) extending parallel to the axis of the trocar.

10. A device according to any one of claims 1 to 9, **characterized in that** the sharp edge (34) of the first end of the trocar (12) lies in a plane that is not orthogonal to the longitudinal axis of the trocar.

11. A device according to any one of claims 1 to 10, **characterized in that** the inner diameter of the first end (32) of the trocar (12) lies in the range 9 mm to 15 mm.
